(19) Europäisches Patentamt
European Patent Office
Office européen des brevets

(11) Veröffentlichungsnummer: **0 445 074 A1**

# (12) EUROPÄISCHE PATENTANMELDUNG

(21) Anmeldenummer: **91810111.4**

(22) Anmeldetag: **20.02.91**

(51) Int. Cl.[5]: **C07D 239/42**, A01N 43/54

(30) Priorität: **27.02.90 CH 628/90**

(43) Veröffentlichungstag der Anmeldung:
**04.09.91 Patentblatt 91/36**

(84) Benannte Vertragsstaaten:
**AT BE CH DE DK ES FR GB GR IT LI LU NL SE**

(71) Anmelder: **CIBA-GEIGY AG**
**Klybeckstrasse 141**
**CH-4002 Basel (CH)**

(72) Erfinder: **Zondler, Helmut, Dr.**
**Oberwilerstrasse 49**
**CH-4103 Bottmingen (CH)**
Erfinder: **Meyer, Alfred, Dr.**
**St. Galler-Ring 220**
**CH-4054 Basel (CH)**
Erfinder: **Riebli, Peter**
**Bienenheim**
**CH-6074 Giswil (CH)**
Erfinder: **Hubele, Adolf, Dr.**
**Obere Egg 9**
**CH-4312 Magden (CH)**

(54) **N-(2,6-Dinitro-3-chlor-4-trifluoromethylphenyl)-4-amino-6-fluorpyrimidin-Derivate und ihre Anwendung als Mikrobizide.**

(57) Verbindungen der Formel

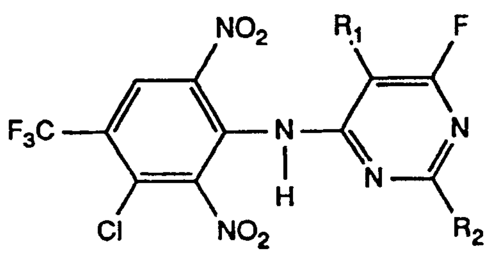

,

in welcher
$R_1$ und $R_2$ unabhängig voneinander Wasserstoff, $C_1$-$C_4$-Alkyl oder Cyclopropyl bedeuten; einschliesslich der Säureadditionssalze der Verbindungen besitzen wertvolle fungizide Eigenschaften. Die neuen Wirkstoffe können im Pflanzenschutz zur Verhütung des Befalls von Kulturpflanzen durch phytopathogene Fungi und zur Bekämpfung dieser Schädlinge eingesetzt werden.

EP 0 445 074 A1

Jouve, 18, rue Saint-Denis, 75001 PARIS

# MIKROBIZIDE

Die vorliegende Erfindung betrifft neue N-(2,6-Dinitro-3-chlor-4-trifluormethylphenyl)-4-amino-6-fluorpyrimidin-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung oder Verhütung eines Befalls von Pflanzen durch phytopathogene Fungi.

Die erfindungsgemässen Verbindungen entsprechen der allgemeinen Formel I

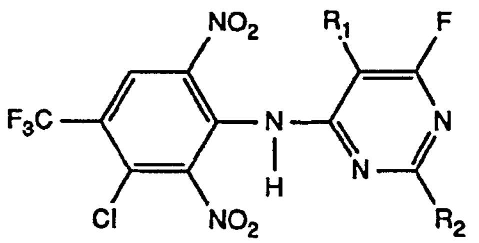

(I),

in welcher
$R_1$ und $R_2$ unabhängig voneinander Wasserstoff, $C_1$-$C_4$-Alkyl oder Cyclopropyl bedeuten; einschliesslich der Säureadditionssalze der Verbindungen der Formel I.

Unter Alkyl sind je nach Anzahl der angegebenen Kohlenstoffatome beispielsweise die folgenden geradkettigen oder verzweigten Gruppen zu verstehen: Methyl, Ethyl, Propyl, Butyl sowie die Isomeren, wie z.B. Isopropyl, Isobutyl, sek.Butyl oder tert.-Butyl.

Die Verbindungen der Formel I sind bei Raumtemperatur Feststoffe, die sich durch sehr wertvolle pflanzenfungizide Eigenschaften auszeichnen. Sie lassen sich daher auf dem Agrogebiet oder verwandten Bereichen zur Bekämpfung von phytopathogenen Fungi einsetzen.

Die Erfindung betrifft sowohl die freien Verbindungen der Formel I als auch deren Additionssalze mit anorganischen und organischen Säuren.

Erfindungsgemässe Salze sind insbesondere Additionssalze mit nach Massgabe des Einsatzzweckes physiologisch unbedenklichen anorganischen oder organischen Säuren, wie beispielsweise Halogenwasserstoffsäuren, z.B. Chlor-, Brom- oder Jodwasserstoffsäure, sowie Schwefelsäure, Phosphorsäure, phosphorige Säure, Salpetersäure, gegebenenfalls halogenierte Fettsäuren, wie Essigsäure, Trichloressigsäure und Oxalsäure, oder Sulfonsäuren, wie Benzolsulfonsäure und Methansulfonsäure oder auch Additionssalze mit geeigneten Salzen, wie beispielsweise Magnesium- oder Calciumchlorid.

Die Verbindungen der Formel I sind neu und stellen als solche eine Stoffauswahl aus den allgemeinen Beschreibungen der europäischen Patentanmeldungen Nr. 139613, Nr. 248 348 und Nr. 248 349 dar. In den vorgenannten Publikationen sind die darin offenbarten Wirkstoffe als Schädlingsbekämpfungsmittel, teilweise auch als Fungizide, beschrieben. Diese Stoffe erfüllen jedoch nicht immer die in der Praxis, insbesondere im Einsatz gegen spezielle Schädlinge, an sie gestellten Forderungen.

Die neuen Wirkstoffe der Formel I dagegen erweisen sich als bevorzugt wirksam gegen spezielle Gattungen der Pilzklassen Fungi imperfecti (z.B. Cercospora), Basidiomyceten (z.B. Puccinia), Ascomyceten (z.B. Erysiphe und Venturia) und Oomyceten (z.B. Plasmopara und Phytophthora). Sie stellen damit im Pflanzenschutz eine wertvolle Ergänzung der Mittel für die Bekämpfung von phytopathogenen Pilzen dar. Für ihre Anwendung in der Praxis besitzen sie vorteilhafterweise sowohl kurative und präventive als auch systemische Eigenschaften und können zum Schutz von zahlreichen Kulturpflanzen eingesetzt werden. Mit diesen Wirkstoffen werden an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet, wobei auch später zuwachsende Pflanzenteile z.B. vor phytopathogenen Pilzen verschont bleiben. Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

In ihrer Wirkungsweise bevorzugte Verbindungen der Formel I sind folgende:
N-(2′,6′-Dinitro-3′-chlor-4′-trifluormethylphenyl)-4-amino-(2,5-diethyl-6-fluor)-pyrimidin;
N-(2′,6′-Dinitro-3′-chlor-4′-trifluormethylphenyl)-4-amino-(5-ethyl-6-fluor)-pyrimidin;
N-(2′,6′-Dinitro-3′-chlor-4′-trifluormethylphenyl)-4-amino-(2-ethyl-5-methyl-6-fluor)-pyrimidin;

N-(2',6'-Dinitro-3'-chlor-4'-trifluormethylphenyl)-4-amino-(2-cyclopropyl-6-fluor)-pyrimidin;
N-(2',6'-Dinitro-3'-chlor-4'-trifluormethylphenyl)-4-amino-(2-n-propyl-6-fluor)-pyrimidin;
N-(2',6'-Dinitro-3'-chlor-4'-trifluormethylphenyl)-4-amino(2-isopropyl-6-fluor)-pyrimidin;
N-(2',6'-Dinitro-3'-chlor-4'-trifluormethylphenyl)-4-amino(5-ethyl-6-fluor)-pyrimidin;
N-(2',6'-Dinitro-3'-chlor-4'-trifluormethylphenyl)-4-amino-(2-cyclopropyl-6-fluor)-pyrimidin;
N-(2',6'-Dinitro-3'-chlor-4'-trifluormethylphenyl)-4-amino-(2,5-dimethyl-6-fluor)-pyrimidin;
N-(2',6'-Dinitro-3'-chlor-4'-trifluormethylphenyl)-4-amino-(2-methyl-5-n-propyl-6-fluor)-pyrimidin;
N-(2',6'-Dinitro-3'-chlor-4'-trifluormethylphenyl)-4-amino-(2-methyl-5-isopropyl-6-fluor)-pyrimidin.

Darunter ist folgende Verbindung als besonders vorteilhaft anzusehen:
N-(2',6'-Dinitro-3'-chlor-4'-trifluormethylphenyl)-4-amino-(2,5-diethyl-6-fluor)-pyrimidin;

Die Verbindungen der Formel I werden hergestellt, indem man umsetzt:
eine Verbindung der Formel II

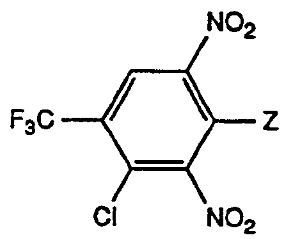

(II)

mit einem Pyrimidin-Derivat der Formel III

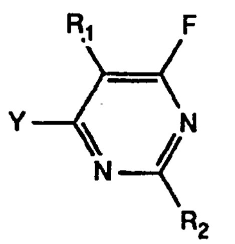

(III),

worin die Substituenten $R_1$ und $R_2$ die unter Formel I angegebenen Bedeutungen besitzen und Z und Y für $NH_2$, Halogen oder $SO_2R$ mit $C_1$-$C_4$-Alkyl oder Aryl für R stehen, wobei falls Z Halogen oder $SO_2R$ darstellt, Y $NH_2$ bedeutet, und falls Z $NH_2$ darstellt, Y Halogen oder $SO_2R$ bedeutet, gegebenenfalls in gegenüber den Reaktionspartnern inerten Lösungsmitteln bei Temperaturen von -80°C bis +150°C, vorzugsweise bei -50°C bis +30°C, gegebenenfalls vorteilhafterweise in Gegenwart von säurebindenden Mitteln.

Als säurebindende Mittel kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Carbonate und Hydrogencarbonate von Alkali- und Erdalkalimetallen sowie Alkaliacetate.

Als reaktionsinerte Lösungs- oder Verdünnungsmittel kommen in Frage beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Die Reaktion der Verbindung der Formel (II) mit der Verbindung der Formel (III) kann auch in einem wässrigen Zweiphasensystem nach dem allgemein bekannten Prinzip der Phasentransferkatalyse durchgeführt werden.

Für die organische, mit Wasser nicht mischbare Phase kommen dabei z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylinethylether usw.. Beispiele geeigneter Phasentransfer-Katalysatoren

sind: Tetraalkylammoniumhalogenid, -hydrogensulfate oder -hydroxid wie Tetrabutylammoniumchlorid, -bromid oder -jodid; Triethylbenzylammoniumchlorid oder -bromid; Cetyltrimethylammoniumchlorid, -bromid oder -jodid; usw.. Als Phasentransfer-Katalysatoren kommen auch Phosphonium-Salze in Betracht. Die Reaktionstemperaturen liegen im allgemeinen zwischen -30° und 130°C bzw. am Siedepunkt des Lösungsmittels oder Lösungsmittelgemisches.

Die Verbindungen der Formeln II und III sind teilweise bekannt oder können nach bekannten Verfahren hergestellt werden [Ser. Khim. Nauk. 1973 (6) 81-85 (CA: 80/59913), Yakugaku Zasshi 87 (11), 1315-1321 (1967) (CA: 68/114540) sowie D.J. Brown; The Chemistry of Heterocyclic Compounds, The Pyrimidines Supplement II 1985, 167].

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kämpfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabeinach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha, bevorzugt 50 g bis 1 kg AS/ha, insbesondere bei 200 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind wasserlösliche 20 bis 250 Aethylenglykoläthergruppen und 10 bis 100 Propylenglykolethergruppen enthaltende Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchliche Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,9 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

1. Herstellungsbeispiele:

1.1 Herstellung von N-(2′,6′-Dinitro-3′-chlor-4′-trifluormethylphenyl)-4-amino-(2,5-diethyl-6-fluor)-pyrimidin (Verb. Nr. 3)

Zu einer Suspension von 5,1 g 2,5-Diethyl-4-amino-6-fluorpyrimidin und 10,1 g 1,3-Dinitro-2,4-dichlor-5-trifluormethylbenzol in 60 ml Tetrahydrofuran wird bei -45°C innerhalb 1/2 Std. eine Lösung von 7,2 g Kalium-tert.-butylat in 50 ml Tetrahydrofuran zugetropft. Nach beendeter Zugabe lässt man die Temperatur des rotgefärbten Reaktionsgemisches langsam auf Raumtemperatur steigen. Nach 12 Std. Rühren bei Raumtemperatur wird das Lösungsmittel unter vermindertem Druck abgedampft und der Rückstand in 100 ml Ethylacetat aufgenommen. Die organische Phase wird 2 mal mit je 150 ml Wasser, 3 mal mit je 150 ml 10%-iger Essigsäure und nochmals 2 mal mit je 100 ml Wasser gewaschen, über Natriumsulfat getrocknet, filtriert und das Lösungsmittel unter Vakuum abgedampft. Der verbleibende Rückstand wird zur Reinigung mittels Hexan/Ethylacetat (4:1 ) über eine Kieselgelsäule chromatographiert. Die Titelverbindung wird in Form weisser Kristalle erhalten; Smp. 111-112°C.

In analoger Weise lassen sich folgende Verbindungen herstellen.

Tabelle 1:

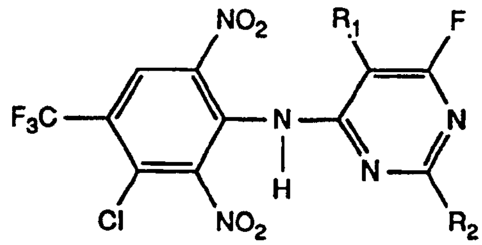

| Nr. | $R_1$ | $R_2$ | physikalische Daten |
|---|---|---|---|
| 1 | H | $CH_3$ | |
| 2 | $CH_3$ | H | |
| 3 | $CH_2CH_3$ | $CH_2CH_3$ | Smp. 111-112°C |
| 4 | 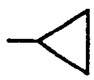 | 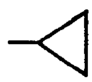 | |
| 5 | $C_4H_9$-n | $C(CH_3)_4$ | |
| 6 | $CH(CH_3)CH_2CH_3$ | $C_3H_7$-i | Smp. 148-150°C |
| 7 | $CH_3$ | $CH_3$ | |
| 8 | H | $C_4H_9$-n | |
| 9 | $CH_2CH_3$ | H | Smp. 79-80°C |
| 10 | 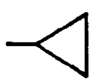 | $CH_2CH_3$ | |
| 11 | $CH_2CH_2CH_3$ | H | |
| 12 | $C_4H_9$-t | $C_4H_9$-n | |
| 13 | $C_3H_7$-i | $CH(CH_3)CH_2CH_3$ | |
| 14 | $CH_2CH_3$ | $C_4H_9$-n | |
| 15 | H | H | |
| 16 | $CH_3$ | $CH_2CH_3$ | Smp. 98-99°C |
| 17 | 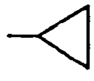 | H | |
| 18 | $C_4H_9$-n | $C_4H_9$-n | Smp. 69-71°C |
| 19 | H | 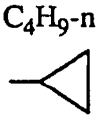 | Smp. 136-138°C |
| 20 | $CH_3$ | $C_3H_7$-n | Smp. 82-86°C |

Tabelle 1: (Fortsetzung)

| Nr. | $R_1$ | $R_2$ | physikalische Daten |
|---|---|---|---|
| 21 | 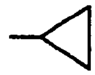 | $CH_3$ | |
| 22 | $CH_2CH_3$ | $C_3H_7$-i | |
| 23 | $C_4H_9$-t | $CH(CH_3)CH_2CH_3$ | |
| 24 | $CH_2CH_2CH_3$ | $CH_2CH_3$ | Smp. 79-82°C |
| 25 | $C_3H_7$-i | 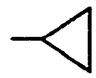 | |
| 26 | $CH_3$ | $C_3H_7$-i | |
| 27 | H | $C_4H_9$-t | |
| 28 | $CH_2CH_3$ | $C_3H_7$-n | Smp. 83-85°C |
| 29 | 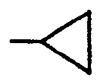 | $C_4H_9$-t | |
| 30 | $CH(CH_3)CH_2CH_3$ | $C_3H_7$-n | |
| 31 | $C_3H_7$-i | $CH_3$ | Smp. 98-100°C |
| 32 | $C_4H_9$-n | H | |
| 33 | $CH_3$ | $C_4H_9$-n | |
| 34 | H | $CH_3$ | |
| 35 | $CH_2CH_3$ | 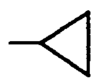 | Smp. 132-134°C |
| 36 | 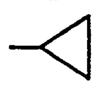 | $C_3H_7$-n | |
| 37 | $CH_2CH_2CH_3$ | 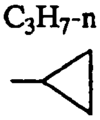 | |
| 38 | $C_4H_9$-t | $C_4H_9$-t | |
| 39 | $CH_2CH_3$ | $CH_3$ | Smp. 118-121°C |
| 40 | $CH_3$ | $CH_2CH(CH_3)_2$ | |
| 41 | H | $C_3H_7$-n | Smp. 130-132°C |
| 42 | 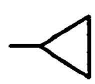 | $C_3H_7$-i | |
| 43 | $CH(CH_3)CH_2CH_3$ | H | |
| 44 | $C_4H_9$-n | $CH(CH_3)CH_2CH_3$ | |

Tabelle 1: (Fortsetzung)

| Nr. | $R_1$ | $R_2$ | physikalische Daten |
|---|---|---|---|
| 45 | $CH_2CH_3$ | $CH_2CH(CH_3)_2$ | |
| 46 | H | $C_3H_7$-i | Smp. 128-129°C |
| 47 | $CH_3$ | 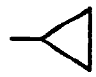 | Smp. 111-113°C |
| 48 | $CH_2CH_2CH_3$ | $C_3H_7$-n | |
| 49 | 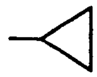 | $C_4H_9$-n | |
| 50 | $C_4H_9$-t | $CH_2CH(CH_3)_2$ | |
| 51 | $C_3H_7$-i | $C_4H_9$-t | |
| 52 | $C_4H_9$-n | 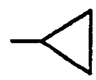 | |
| 53 | $CH_3$ | $CH(CH_3)CH_2CH_3$ | |
| 54 | H | $CH_2CH(CH_3)_2$ | |
| 55 | $CH_2CH_3$ | $C(CH_3)_4$ | |
| 56 | $CH(CH_3)CH_2CH_3$ | $CH_3$ | |
| 57 | 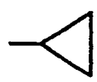 | $CH_2CH(CH_3)_2$ | |
| 58 | $CH_2CH_2CH_3$ | $CH(CH_3)_2$ | |
| 59 | $C_4H_9$-t | $C_3H_7$-n | |
| 60 | $C_3H_7$-i | $CH_2CH(CH_3)_2$ | |
| 61 | $CH_2CH(CH_3)_2$ | H | |
| 62 | H | $CH(CH_3)CH_2CH_3$ | |
| 63 | $CH_3$ | $C_4H_9$-n | |
| 64 | $C_4H_9$-n | $CH_2CH(CH_3)_2$ | |
| 65 | $CH_2CH(CH_3)_2$ | $CH_2CH_3$ | |
| 66 | $CH_2CH_3$ | $CH(CH_3)CH_2CH_3$ | |
| 67 | $C_4H_9$-t | $C_3H_7$-i | |
| 68 | $CH_2CH_2CH_3$ | $C_4H_9$-n | |

Tabelle 1: (Fortsetzung)

| Nr. | $R_1$ | $R_2$ | physikalische Daten |
|---|---|---|---|
| 69 | $C_3H_7$-i | $C_4H_9$-n | |
| 70 | 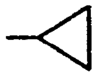 | $CH(CH_3)CH_2CH_3$ | |
| 71 | $CH(CH_3)CH_2CH_3$ | 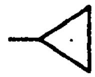 | |
| 72 | $C_4H_9$-t | 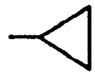 | |
| 73 | $CH_2CH_2CH_3$ | $CH_3$ | Smp. 95-97°C |
| 74 | $CH(CH_3)CH_2CH_3$ | $C_4H_9$-n | |
| 75 | $C_4H_9$-n | $C_3H_7$-i | |
| 76 | $C_3H_7$-i | $C_3H_7$-i | |
| 77 | $C_4H_9$-t | $CH_3$ | |
| 78 | $CH_2CH(CH_3)_2$ | $C_3H_7$-n | |
| 79 | $CH(CH_3)CH_2CH_3$ | $CH(CH_3)CH_2CH_3$ | |
| 80 | $C_4H_9$-n | $C_3H_7$-n | |
| 81 | $CH_2CH(CH_3)_2$ | $C_3H_7$-i | |
| 82 | $CH_2CH_2CH_3$ | $CH_2CH(CH_3)_2$ | |
| 83 | $C_3H_7$-i | $C_3H_7$-n | |
| 84 | $CH_2CH(CH_3)_2$ | $C_4H_9$-n | |
| 85 | $C_4H_9$-t | $CH_2CH_3$ | |
| 86 | $CH_2CH(CH_3)_2$ | $CH_2CH(CH_3)_2$ | |
| 87 | $CH_2CH(CH_3)_2$ | $C_4H_9$-t | |
| 88 | $CH_2CH_2CH_3$ | $CH(CH_3)CH_2CH_3$ | |
| 89 | $C_4H_9$-n | $CH_3$ | Smp. 130-132°C |
| 90 | $CH(CH_3)CH_2CH_3$ | $C_4H_9$-t | |
| 91 | $CH_2CH(CH_3)_2$ | 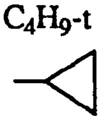 | |
| 92 | $C_3H_7$-i | $CH_2CH_3$ | |

Tabelle 1: (Fortsetzung)

| Nr. | $R_1$ | $R_2$ | physikalische Daten |
|---|---|---|---|
| 93 | $CH_2CH(CH_3)_2$ | $CH(CH_3)CH_2CH_3$ | |
| 94 | $C_4H_9$-t | H | |
| 95 | $CH_2CH_2CH_3$ | $C_4H_9$-t | |
| 96 | $CH(CH_3)CH_2CH_3$ | $CH_2CH(CH_3)_2$ | |
| 97 | $C_4H_9$-n | $CH_2CH_3$ | |
| 98 | $CH(CH_3)CH_2CH_3$ | $CH_2CH_3$ | |
| 99 | $C_3H_7$-i | H | |
| 100 | $CH_2CH(CH_3)_2$ | $CH_3$ | |

2. Formulierungsbeispiele für feste Wirkstoffe der Formel I (% = Gewichtsprozent)

| 2.1. Spritzpulver | a) | b) | c) |
|---|---|---|---|
| Wirkstoff aus der Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.2. Emulsions-Konzentrat

| | |
|---|---|
| Wirkstoff aus der Tabelle 1 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.3. Stäubemittel | a) | b) |
|---|---|---|
| Wirkstoff aus der Tabelle 1 | 5 % | 8 % |
| Talkum | 95 % | - |
| Kaolin | - | 92 % |

Man erhält anwendungsfertige Stäubemittel, in dem der Wirkstoff mit dem Träger vermischt auf einer geeigneten Mühle vermahlen wird.

2.4. Extruder Granulat

| | |
|---|---|
| Wirkstoff aus der Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5. Umhüllungs-Granulat

| | |
|---|---|
| Wirkstoff aus der Tabelle 1 | 3 % |
| Polyethylenglykol (MG 200) | 3 % |
| Kaolin | 94 % |

(MG = Molekulargewicht)

Der fein gemahlene Wirkstoff wird in einem Mis cher auf das mit Polyethylenglykol angefeuchtete Kaolin gleichmässig aufgetragen. Auf diese Weise erhält man staubfreie Umhüllungs-Granulate.

2.6. Suspensions-Konzentrat

| | |
|---|---|
| Wirkstoff aus der Tabelle 1 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95- 10 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) Systemische Wirkung

Zu Weizenpflanzen werden 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus Tabelle 1 zeigen gegen Puccinia-Pilze eine sehr gute Wirkung. Unter anderen hemmte die Verbindung Nr. 46 den Puccinia-Befall auf 0 bis 5 %. Unbehandelte aber infizierte Kontrollpflanzen wiesen dagegen einen Puccinia-Befall von 100 % auf.

Beispiel 3.2: Wirkung gegen Cercospora arachidicola auf Erdnusspflanzen

Residual-protektive Wirkung

10-15 cm hohe Erdnusspflanzen werden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt.
Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigen Erdnusspflanzen, die mit Wirkstoffen aus Tabelle 1 behandelt werden, einen stark reduzierten Cercospora-Befall. So verhinderten die Verbindungen Nr. 3, 9, 16, 19 und 46 in obigen Versuchen das Auftreten von Flecken fast vollständig (0-10 %).

Beispiel 3.3: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus Tabelle 1 zeigen gute Wirkung gegen den Erysiphae-Pilz. So hemmten die Verbindungen Nr. 3, 16 und 46 den Pilzbefall bei Gerste auf weniger als 10 %. Unbehandelte, aber infizierte Kontrollpflanzen zeigten dagegen einen Erysiphae-Befall von 100 %.

Beispiel 3.4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tage bei 90- 100 % relativer Luftfeuchtigkeit inkubiert und während 10 weiterer Tage in einem Gewächshaus bei 20-24°C aufgestellt. Der Schorfbefall wird 15 Tage nach der Infektion beurteilt. Verbindungen aus Tabelle 1 zeigen einen stark reduzierten Krankheitsbefall. So hemmten die Verbindungen Nr. 3, 9, 16, 19, 41 und 46 den Venturia-Befall auf 5 %. Unbehandelte aber infizierte Kontrolltriebe wurden dagegen 100%-ig befallen.

Beispiel 3.5: Wirkung gegen Botrytis cinerea auf Bohnen

Residual protektive Wirkung

Ca. 10 cm hohe Bohnen-Pflanzen werden mit einer aus Spritzpulver des Wirkstoffes hergestelten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21°C erfolgte die Beurteilung des Pilzbefalls. Verbindungen aus Tabelle 1 hemmten die Pilzinfektion sehr stark. Der Botrytis-Befall unbehandelter aber infizierter Bohnenpflanzen betrug 100 %.

Beispiel 3.6: Wirkung gegen Phytophthora infestans auf Tomatenpflanzen

a) Residual-protektive Wirkung

Tomatenpflanzen werden in 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

b) Systemische Wirkung

Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

In obigen Versuchen zeigen die Verbindungen aus Tabelle 1 eine sehr gute Wirkung gegen Phytophthora. Im Vergleich zu unbehandelten, aber infizierten Kontrollpflanzen mit 100 % Befall bewirkten die Verbindungen Nr. 3, 9, 19, 41 und 49 eine fast vollständige Unterdrückung des Pilzbefalls (0 bis 10 %).

Beispiel 3.7: Wirkung gegen Plasmopara viticola auf Reben

a) Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

b) Residual-kurative Wirkung

Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20°C werden die infizierten Pflanzen getrocknet und mit einer aus Spritzpulver des Wirkstoffes hergestellten Spritzbrühe (0,006 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus Tabelle 1 zeigen gegen Plasmopara viticola auf Reben eine sehr gute Wirkung, insbesondere die Verbindungen Nr. 9, 19, 41 und 46 bewirkten eine vollständige Unterdrückung des Pilzbefalls (0 bis 5 %).

**Patentansprüche**

1. Verbindungen der Formel I

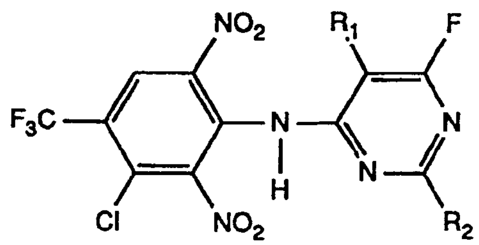

(I),

in welcher
$R_1$ und $R_2$ unabhängig voneinander Wasserstoff, $C_1$-$C_4$-Alkyl oder Cyclopropyl bedeuten; einschliesslich der Säureadditionssalze der Verbindungen der Formel I.

2. Eine Verbindung der Formel I gemäss Anspruch 1 aus der Gruppe:
N-(2′,6′-Dinitro-3′-chlor-4′-trifluormethylphenyl)-4-amino-(2,5-diethyl-6-fluor)-pyrimidin;
N-(2′,6′-Dinitro-3′-chlor-4′-trifluormethylphenyl)-4-amino-(5-ethyl-6-fluor)-pyrimidin;
N-(2′,6′-Dinitro-3′-chlor-4′-trifluormethylphenyl)-4-amino-(2-ethyl-5-methyl-6-fluor)-pyrimidin;
N-(2′,6′-Dinitro-3′-chlor-4′-trifluormethylphenyl)-4-amino-(2-cyclopropyl-6-fluor)-pyrimidin;
N-(2′,6′-Dinitro-3′-chlor-4′-trifluormethylphenyl)-4-amino-(2-n-propyl-6-fluor)-pyrimidin;
N-(2′,6′-Dinitro-3′-chlor-4′-trifluormethylphenyl)-4-amino-(2-isopropyl-6-fluor)-pyrimidin;
N-(2′,6′-Dinitro-3′-chlor-4′-trifluormethylphenyl)-4-amino-(5-ethyl-6-fluor)-pyrimidin;
N-(2′,6′-Dinitro-3′-chlor-4′-trifluormethyl)phenyl-4-amino-(2-cyclopropyl-6-fluor)-pyrimidin;
N-(2′,6′-Dinitro-3′-chlor-4′-trifluormethylphenyl)-4-amino-(2,5-dimethyl-6-fluor)-pyrimidin;
N-(2′,6′-Dinitro-3′-chlor-4′-trifluormethylphenyl)-4-amino-(2-methyl-5-n-propyl-6-fluor)-pyrimidin;
N-(2′,6′-Dinitro-3′-chlor-4′-trifluormethylphenyl)-4-amino-(2-methyl-5-isopropyl-6-fluor)-pyrimidin.

3. N-(2′,6′-Dinitro-3′-chlor-4′-trifluormethylphenyl)-4-amino-(2,5-diethyl-6-fluor)-pyrimidin.

4. Verfahren zur Herstellung von Verbindungen der Formel I in Anspruch 1, dadurch gekennzeichnet, dass man umsetzt:
eine Verbindung der Formel II

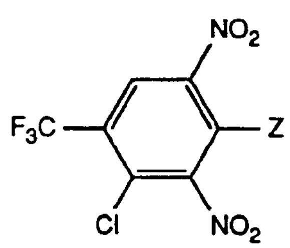

(II)

mit einem Pyrimidin-Derivat der Formel III

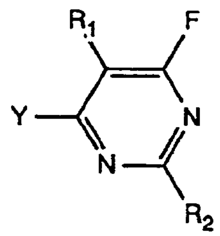

(III),

worin die Substituenten $R_1$ und $R_2$ die unter Formel I angegebenen Bedeutungen besitzen und Z und Y für $NH_2$, Halogen oder $SO_2R$ mit $C_1$-$C_4$-Alkyl oder Aryl für R stehen, wobei falls Z Halogen oder $SO_2R$ darstellt, Y $NH_2$ bedeutet und falls Z $NH_2$ darstellt, Y Halogen oder $SO_2R$ bedeutet, bei Temperaturen von -80°C bis +150°C.

5. Mittel zur Bekämpfung oder Verhütung eines Befalls von Pflanzen durch phytopathogene Fungi, dadurch gekennzeichnet, dass es als aktive Komponente mindestens eine Verbindung der Formel I gemäss Anspruch 1 zusammen mit einem geeigneten Trägermaterial enthält.

6. Mittel gemäss Anspruch 5, dadurch gekennzeichnet, dass es als aktive Komponente mindestens eine Verbindung der Formel I von Anspruch 2 enthält.

7. Mittel gemäss Anspruch 5, dadurch gekennzeichnet, dass es als aktive Komponente mindestens eine Verbindung der Formel I von Anspruch 3 enthält.

8. Verfahren zur Bekämpfung oder Verhütung eines Befalls von Kulturpflanzen durch phytopathogene Fungi, dadurch gekennzeichnet, dass man als Wirkstoff eine Verbindung der Formel I gemäss Anspruch 1 auf die Pflanze, auf Pflanzenteile oder ihren Standort appliziert.

9. Verfahren gemäss Anspruch 8, dadurch gekennzeichnet, dass man als Wirkstoff eine Verbindung in einem der Ansprüche 2 und 3 appliziert.

10. Verfahren zur Herstellung eines agrochemischen Mittels in Anspruch 5, dadurch gekennzeichnet, dass man mindestens eine Verbindung der Formel I in Anspruch 1 mit geeigneten festen oder flüssigen Zusatzstoffen und/oder Tensiden innig vermischt.

**Patentansprüche Für folgenden Vertragsstaat: ES**

1. Verfahren zur Herstellung von Verbindungen der Formel I

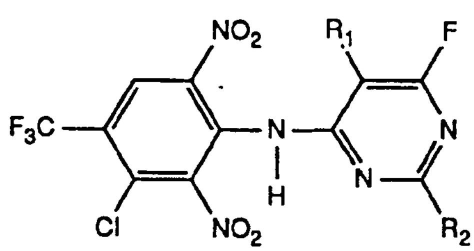

(I),

in welcher
$R_1$ und $R_2$ unabhängig voneinander Wasserstoff, $C_1$-$C_4$-Alkyl oder Cyclopropyl bedeuten; einschliesslich der Säureadditionssalze der Verbindungen der Formel I, dadurch gekennzeichnet, dass man umsetzt: eine Verbindung der Formel II

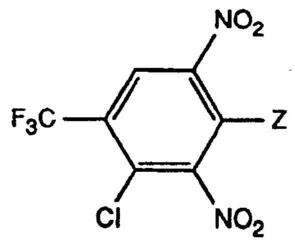

(II)

mit einem Pyrimidin-Derivat der Formel III

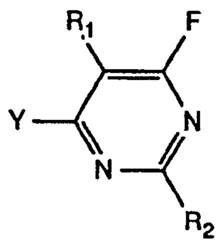

(III),

worin die Substituenten $R_1$ und $R_2$ die unter Formel I angegebenen Bedeutungen besitzen und Z und Y für $NH_2$, Halogen oder $SO_2R$ mit $C_1$-$C_4$-Alkyl oder Aryl für R stehen, wobei falls Z Halogen oder $SO_2R$ darstellt, Y $NH_2$ bedeutet, und falls Z $NH_2$ darstellt, Y Halogen oder $SO_2R$ bedeutet, bei Temperaturen von -80°C bis +150°C.

2. Verfahren gemäss Anspruch 1, dadurch gekennzeichnet, dass man eine Verbindung aus der Gruppe:
N-(2',6'-Dinitro-3'-chlor-4'-trifluormethylphenyl)-4-amino-(2,5-diethyl-6-fluor)-pyrimidin;
N-(2',6'-Dinitro-3'-chlor-4'-trifluormethylphenyl)-4-amino-(5-ethyl-6-fluor)-pyrimidin;
N-(2',6'-Dinitro-3'-chlor-4'-trifluormethylphenyl)-4-amino-(2-ethyl-5-methyl-6-fluor)-pyrimidin;
N-(2',6'-Dinitro-3'-chlor-4'-trifluormethylphenyl)-4-amino-(2-cyclopropyl-6-fluor)-pyrimidin;
N-(2',6'-Dinitro-3'-chlor-4'-trifluormethylphenyl)-4-amino-(2-n-propyl-6-fluor)-pyrimidin;
N-(2',6'-Dinitro-3'-chlor-4'-trifluormethylphenyl)-4-amino-(2-isopropyl-6-fluor)-pyrimidin;
N-(2',6'-Dinitro-3'-chlor-4'-trifluormethylphenyl)-4-amino-(5-ethyl-6-fluor)-pyrimidin;
N- (2',6'-Dinitro-3'-chlor-4'-trifluormethylphenyl)-4-amino-(2-cyclopropyl-6-fluor)-pyrimidin;
N-(2',6'-Dinitro-3'-chlor-4'-trifluormethylphenyl)-4-amino-(2,5-dimethyl-6-fluor)-pyrimidin;
N-(2',6'-Dinitro-3'-chlor-4'-trifluormethylphenyl)-4-amino-(2-methyl-5-n-propyl-6-fluor)-pyrimidin;
N-(2',6'-Dinitro-3'-chlor-4'-trifluormethylphenyl)-4-amino-(2-methyl-5-isopropyl--6-fluor)-pyrimidin;
herstellt.

3. Verfahren gemäss Anspruch 1, dadurch gekennzeichnet, dass man die Verbindung N-(2',6'-Dinitro-3'-chlor-4'-trifluormethylphenyl)-4-amino-(2,5-diethyl-6-fluor)-pyrimidin herstellt.

4. Mittel zur Bekämpfung oder Verhütung eines Befalls von Pflanzen durch phytopathogene Fungi, dadurch gekennzeichnet, dass es als aktive Komponente mindestens eine Verbindung der Formel I von Anspruch 1 zusammen mit einem geeigneten Trägermaterial enthält.

5. Mittel gemäss Anspruch 4, dadurch gekennzeichnet, dass es als aktive Komponente mindestens eine Verbindung der Formel I von Anspruch 2 enthält.

6. Mittel gemäss Anspruch 4, dadurch gekennzeichnet, dass es als aktive Komponente mindestens eine Verbindung der Formel I von Anspruch 3 enthält.

Europäisches Patentamt

# EUROPÄISCHER RECHERCHENBERICHT

Nummer der Anmeldung

EP 91 81 0111

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y,D | EP-A-248349 (HOECHST AG)<br>* Ansprüche 1, 3-8 *<br>* Tabelle 1, Verbindung 1.186 *<br>--- | 1, 4-10 | C07D239/42<br>A01N43/54 |
| Y,D | EP-A-248348 (HOECHST AG)<br>* Ansprüche 1, 4, 5, 8, 9 *<br>* Tabelle 6, Verbindung 6.1 *<br>--- | 1, 4-10 | |
| Y,D | EP-A-139613 (CIBA-GEIGY AG)<br>* Ansprüche 1-5, 7, 8, 10-12*<br>* Tabelle 2, Verbindung 2.2; Tabelle 6, Verbindungen 6.9-10 *<br>--- | 1, 4-10 | |
| A | EP-A-126254 (CIBA-GEIGY AG)<br>* Ansprüche 1, 12-18 *<br>----- | 1, 4-10 | |

RECHERCHIERTE SACHGEBIETE (Int. Cl.5 )

C07D239/00
A01N43/00

Der vorliegende Recherchenbericht wurde für alle Patentansprüche erstellt

| Recherchenort | Abschlußdatum der Recherche | Prüfer |
|---|---|---|
| BERLIN | 16 MAI 1991 | VAN AMSTERDAM L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)